(19) Europäisches Patentamt

European Patent Office

Office européen des brevets

(11) Publication number: **0 196 205**
**A2**

(12) # EUROPEAN PATENT APPLICATION

(21) Application number: **86302135.8**

(22) Date of filing: **24.03.86**

(51) Int. Cl.⁴: **G 01 N 15/02, G 01 N 33/28, B 03 C 1/00**

(30) Priority: **27.03.85 US 716732**

(43) Date of publication of application: **01.10.86 Bulletin 86/40**

(84) Designated Contracting States: **DE FR GB IT NL**

(71) Applicant: **THE STANDARD OIL COMPANY, 200 Public Square, 36-F-3454, Cleveland Ohio 44114-2375 (US)**

(72) Inventor: **Laskowski, John Stanley, 5821 Sequoia Drive, Parma Ohio 44134 (US)**

(74) Representative: **Chariton, Peter John et al, Elkington and Fife High Holborn House 52/54 High Holborn, London WC1V 6SH (GB)**

(54) **Improved apparatus for segregating particulate matter.**

(57) An improved apparatus is provided for the separation of particles from a fluid medium in accordance with particle size wherein a reservoir (26) which holds a measured amount of the particle-containing fluid (22) is disposed anterior to a length of tubing (24) which terminates over a substrate (20). Particles in the fluid are not subjected to any external forces which may modify or alter the particles during the analysis process.

EP 0 196 205 A2

(84-P-1474)

0196205

# IMPROVED APPARATUS FOR SEGREGATING PARTICULATE MATTER

This invention relates to an apparatus for the detection of hyperfine particles in a fluid and the optical determination of the type and size distribution of such particles. Such an apparatus is useful in the analysis of wear of moving parts, such as engine bearings and to the testing of the performance of various lubricating materials.

The use of magnetic techniques for separating ferromagnetic materials from background substances is well known. Recent refinements of such techniques have made it possible to precipitate hyperfine ferromagnetic wear particles from a lubricant taken from a machine, such as a diesel engine, to determine the wear condition of the machine by optical analysis of such particles. A detailed description of an apparatus and procedure for performing such precipitation and analysis is set forth in United States patent no. 4,047,814, entitled "Method and Apparatus for Segregating Particulate Matter", to Westcott.

In accordance with that prior disclosure, a particle-containing lubricant sample taken from a piece of equipment is caused to flow along a shallow channel in a nearly horizontal glass substrate positioned over a magnet, the air gap of which is aligned with the longitudinal axis of the substrate. The magnet develops a magnetic field having a very high gradient perpendicular to the substrate surface along the lubricant flow path on the substrate. Ferromagnetic wear particles are drawn by magnetic force down through the lubricant liquid so as to precipitate onto the substrate surface.

In carrying out this procedure with ferromagnetic wear particles, the larger particles are precipitated first and the smaller particles are precipitated further along the flow path. Analysis of the relative proportions of large and small size wear particles provides significant information about the state of wear of the machine from which the lubricant sample was taken. Similar techniques can be used to precipitate non-magnetic particles which wear against a ferris metal such as steel, since the ferris metal smears on or become embedded in the non-magnetic material which thus becomes effectively magnetic.

In addition, Westcott, et al., in United States patent no. 4,187,170 entitled "Magnetic Techniques for Separating Non-Magnetic Materials" discloses a process for magnetically separating non-magnetic materials from a mixture by combining the mixture with a magnetizing solution containing the salt of a magnetic element. The magnetic atoms attach to available sites on the molecules of the non-magnetic material so as to develop in that material a positive susceptibility to magnetism. In this way, non-magnetic materials may be utilized in the apparatus taught above in United States patent no. 4,047,814, and so, examined in a similar manner.

The apparatus taught by Westcott above, is a manually operated apparatus requiring operator attention during the full course of the analysis. In addition, the lubricant may have to travel a rather long and tortuous path, including passage through a pump, which may possibly alter the metal particles in the lubricant and so yield an inaccurate or misleading particle size analysis.

What is needed in the area of an apparatus for segregating particulate matter is an apparatus that does not have to be attended at all times and which does not subject the fluid to be analyzed to any conditions which may alter the particles suspended in the fluid.

0196205

Accordingly, it is one object of the present invention to provide an improved apparatus for the segregation of particulate matter.

It is another object of the present invention to provide an improved apparatus for analyzing a fluid to determine the concentration of hyperfine magnetic particles therein which apparatus is semiautomatic.

It is yet another object of this invention to provide an apparatus for the segregation of particulate matter, which apparatus does not alter the particles to be segregated during such segregation process.

These and other objects of the present invention will become apparent to one skilled in the art from the description of the invention below and from the appended claims.

The present invention relates to an apparatus for the separation of particles from a fluid medium in accordance with particle size and for use with a magnet providing an inhomogeneous field, which apparatus comprises a substrate positioned in the magnetic field and supporting fluid flow of the particle-containing fluid over a surface thereof and receiving particles from the fluid thereon, and tubing for transporting said fluid to said substrate, characterised by:

a reservoir disposed anterior to said tubing, which reservoir contains a predetermined amount of the particle-containing fluid and means for positively transfering the particle-containing fluid into said tubing and there onto said substrate.

It has been found that prior methods for analysis of particles in lubricants carried out in such a manner as to permit a visual inspection of the particles are an excellent source for determination of machinery wear. To date, such methods and apparatus have been labor intensive. requiring the presence of an operator for the duration of the analysis. In addition, the operator is generally restricted to the performance of one analysis at a time. In accordance with the present invention, a reservoir system is provided at the anterior end of tubing that terminates on the substrate over which the fluid passes and deposits its particulate matter. The reservoir holds a predetermined amount of fluid. Once the apparatus in accordance with this invention has been started, it is then possible to leave the apparatus for the duration of the run. Additionally, several reservoirs may be prepared simultaneously with fluid, and thereafter be quickly disposed in the apparatus and operated therefrom.

By providing reservoirs anterior to the short length of tubing that terminates at the receiving end of the substrate, the particles in the particle-containing fluid are maintained in the same condition in which they existed when the sample was first taken. Extensive travel paths and passage through equipment such as pumps, that may alter the shape and/or size of the particles, are avoided, thereby evading possible misleading analyses.

The improved apparatus of this invention is used in the known process for analyzing hyperfine particulate substances in a fluid by collecting the particles and classifying them in particulate form. The particles are generally examined visually with the aid of a microscope. Such characteristics as particle size and size distribution, particle shape and, to some extent, even the chemical make-up of individual particles provides information that is important in analyzing the wear of moving parts, as in heavy industrial machinery. The particle-containing fluid is flowed over a collecting substrate in the presence of a magnetic or electric field having an intense gradient. The field causes magnetically susceptible or electrically polarizable particles, as the case may be, to settle on the substrate at a rate which is proportional to their concentration in the fluid, as well as proportional to the flow rate of the fluid over the substrate. In this manner, the concentration of particles can be determined. The particles are also deposited on the substrate in accordance with their size, the larger particles being deposited first, and smaller particles being deposited later.

When a viscous fluid such as engine oil is being examined, the collection of the particles onto the substrate under the influence of the magnetic or electric field may be promoted by adding a solvent to the oil. The solvent loosens the bonds between the particulate matter and the oil, or additives in the oil, and thus promotes faster particle precipitation. After the appropriate amount of fluid has been flowed over the substrate, the latter is then preferably washed with a wash solvent that removes the remaining liquid from the surface of the substrate and leaves the particles attached to the surface.

After the liquid and solvent have been evaporated from the slide, an examination of the particles is conducted with a microscope having a high numerical aperture, such as 0.65 or greater. It has been found that color illumination enables one to observe characteristics of the precipitate which are not otherwise readily observable. For example, one can distinguish metals from their oxides or sulfides even when the particles being examined are less then a micron in size by utilizing red and green filters.

By the use of this technique, one can test or check the performance of various lubricants as well as monitor equipment for the purpose of scheduling servicing, rather than servicing equipment that does not require such servicing or equipment that has already failed.

The improved apparatus disclosed herein may utilize separate reservoirs and tubing for dispensing the fluid to be analyzed and the wash solvent. In addition, this apparatus may be provided in such a manner as to become automatic, that is after inserting a reservoir holding the particle-containing fluid and a second reservoir holding the wash solvent into the apparatus, the operator may begin the apparatus' operation which then automatically dispenses the particle-containing fluid from the first reservoir onto the substrate and then dispenses the wash solvent from the second reservoir onto the substrate. This may be accomplished by pressurizing the reservoirs to provide a positive dispensation of fluid into the adjacent tubing and there onto the substrate. Such pressure may be provided as, for example, from gas pressure or by mechanical pressure such as a piston inserted into a reservoir to provide a positive displacement of fluid into the tubing.

In one embodiment of this apparatus, a positive displacement pump, which may be variably adjusted for speed, is utilized to insert a first piston into a first reservoir and dispense a particle-containing fluid therefrom onto a substrate, and then to insert a second piston into a second reservoir which disposes a wash solvent over the same substrate.

The foregoing and other objects and features of the invention will be more readily understood with reference to the following description in conjunction with the accompanying drawings in which:

Figure 1 is a pictorial view of one embodiment of an apparatus in accordance with this invention; and

Figure 2 is a pictorial view of a second embodiment of an apparatus in accordance with the present invention.

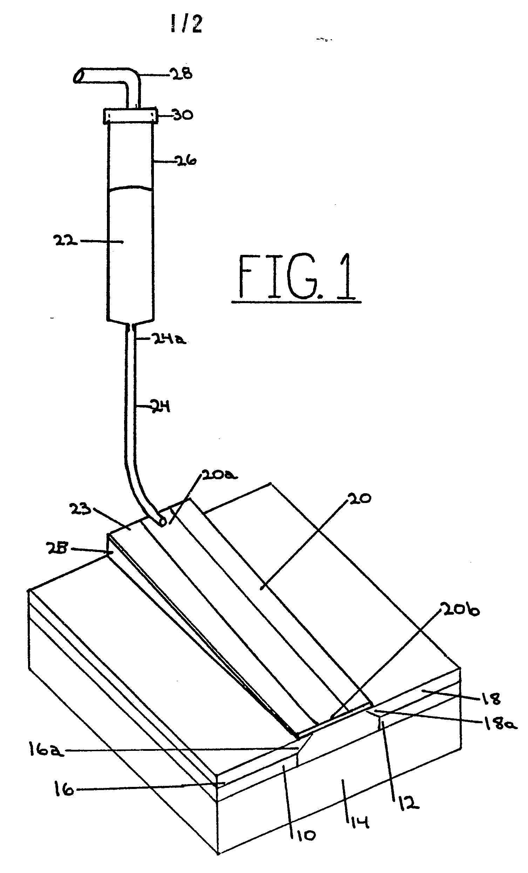

In Figure 1, a pair of magnets, 10 and 12 are positioned on a magnetic return 14 and have pole pieces, 16 and 18, respectively, associated with them. The pole pieces have leading edges, 16a and 18a, respectively, which are separated by a narrow air gap so as to produce an intense field having a very high gradient in the air gap between them. This gradient is in a plane transverse to the pole piece edges and has a downwardly directed component which magnetically collects particles onto a substrate 20 positioned above the magnets.

A fluid 22, shown disposed in reservoir 26, containing particles to be segregated is flowed over the substrate 20 at a fixed rate. The fluid is supplied to a receiving end 20a of the substrate by a length of tubing 24 and drained from a discharge end 20b of the substrate by another length of tubing or by flowing into a collection receptacle, not shown in the figure.

Tubing 24 is attached, at its anterior end 24a to a reservoir 26 in accordance with the present invention. The reservoir 26 is, preferably, removable from the remainder of the apparatus, and so may be filled with a predetermined amount of fluid at a site removed from the apparatus. Several reservoirs may be filled with fluid and set aside for subsequent particle segregation. This is especially useful in instances where reservoirs may be taken into a plant to obtain fluid samples from operating machinery and then returned to the site of the apparatus for processing. As will be further discussed below, reservoirs may also be filled with a desired amount of wash solvent to be dispensed through tubing to wash the particles fixed on the substrate.

The reservoir 26 is positioned in such a manner that pressure may be positively applied to the fluid therein, directing the particle-containing fluid 22 through the tube 24 and onto the substrate 20. As depicted in Figure 1, this pressure may come from a gas provided by a pressurizing source, not shown, and directed through outlet 28 into the reservoir 26 that is sealed, as by means of closure 30. The pressure applied to the reservoir may be held constant or may be varied, as is necessary to obtain reliable results for various samples to be analyzed.

This apparatus reduces the amount of movement and stress placed on the particle-containing fluid to be evaluated. The particle-containing fluid 22 may be collected from its working environment directly into a reservoir 26, which is then returned to the apparatus, inserted therein and passed through a short length of tubing 24 onto the examining substrate 20. The fluid and particles therein are not likely to be altered by this apparatus, which alteration may otherwise yield false and/or misleading wear indications.

The substrate is typically formed from a thin translucent base such as glass, on the order of about 0.076mm in thick. Strips of non-wettable material such as Teflon maybe secured to the edges of the base to form a flow-defining channel on the surface of the base.

The substrate 20 is centered over the air gap between the pole pieces 16 and 18 and is inclined at a slight angle, on the order of about 1°, to the plane of the edges 16a and 18a of the pole pieces by means of a wedge 28, for example, which elevates the fluid-receiving end 20a of the substrate above the discharge end 20b. This causes the magnetic field to increase along the substrate in the longitudinal direction, since the receiving end 20a is positioned farther from the pole pieces than the discharge end 20b. Thus, as the fluid 22 travels down the substrate, the magnetically susceptible particles in it are subjected to an increasingly intense magnetic field gradient. This assists in segregating the particles in the fluid according to size.

Fluid traveling down the substrate is subjected to an increasingly intense magnetic field. The larger particles have a larger ratio of volume-to-surface area than the smaller particles; consequently, viscous drag, which is proportional to surface area, is of lesser magnitude per unit volume for the large particles than for the smaller particles. At the same time, magnetization of the particles is roughly proportional to particle size, and so the larger particles precipitate out first, even though they are subjected to a lesser field and gradient. In consequence, the particles are distributed along the length of the substrate in accordance with their size, the larger particles precipitating out primarily at the receiving end 20a of the substrate, the smaller particles precipitating out primarily at the discharge end 20b. Intermediate sized particles are deposited in-between. Thus, the present invention provides a ready means for determining not only the rate of deposit of the particles, but also their size distribution in the fluid.

In flowing over the substrate, the fluid being analyzed is characterized by a velocity which ranges from zero at the substrate surface to a maximum at the uppermost surface of the fluid. As particles are drawn down toward the substrate, they penetrate flow layers of successively decreasing velocity and thus their longitudinal velocity along the substrate decreases. At the same time, they continue to be accelerated in the vertical direction by the magnetic forces. Accordingly, their trajectories become more vertical until ultimately the particles impact the substrate surface at a nearly vertical angle. The larger particles are drawn to the substrate first because of their larger volume-to-area ratio and thus are deposited at the receiving end of the substrate. The smaller particles, characterized as they are by a smaller volume-to-area ratio, are deposited further down the substrate.

The time required to prepare a substrate suitable for examination varies with such factors as the viscosity of the fluid to be analyzed, the particle concentration in the fluid, the intensity of the magnetic field and it's gradient, and the sensitivity of the viewing apparatus, among other factors. Further, in a fluid such as engine oil, various additives are often mixed with the oil to cause the oil to adhere to metal better. These additives generally adhere to the metal particles
and cause the oil in the immediate vicinity around the particles to gel,
thereby maintaining the particles in solution longer than would otherwise
occur. To decrease the time required to obtain an appropriate sample,
therefore, it is sometimes advantageous to add a solvent to the
particle-containing fluid which frees the particles from the oil
globules, thereby reducing particle drag and viscosity, and accelerating
precipitation of particles onto the sub- strate. In the case of engine
oil, perchloroethylene is a preferred solvent.

All traces of oil from the slide are removed by flushing the
substrate with wash solvent. This occurs after the particles have been
precipitated from the fluid onto the substrate. The substrate is then
allowed to dry thoroughly, after which it will generally be found that
the particles rigidly adhere to the substrate.

The wash solvent is separately flowed over the particles
disposed on the substrate by dispensing the wash solvent separately in a
second reservoir, not shown in Figure 1, and replacing the
particle-containing fluid reservoir with the wash solvent reservoir. The
wash solvent is then dispensed through tube 24 and onto the substrate 20
in the same manner in which the particle-containing fluid was first
dispensed through the tube and onto the substrate.

The reservoirs utilized for containing the particle-containing
fluid and wash solvent may be dispensable, as may also be the tubing that
transports the fluid and solvent from the reservoirs to the substrate.
In this manner contamination from sample-to-sample is avoided.

The apparatus depicted in Figure 1 requires the attention of an
operator to replace the particle-containing fluid reservoir with a wash
solvent reservoir midway through the procedure.

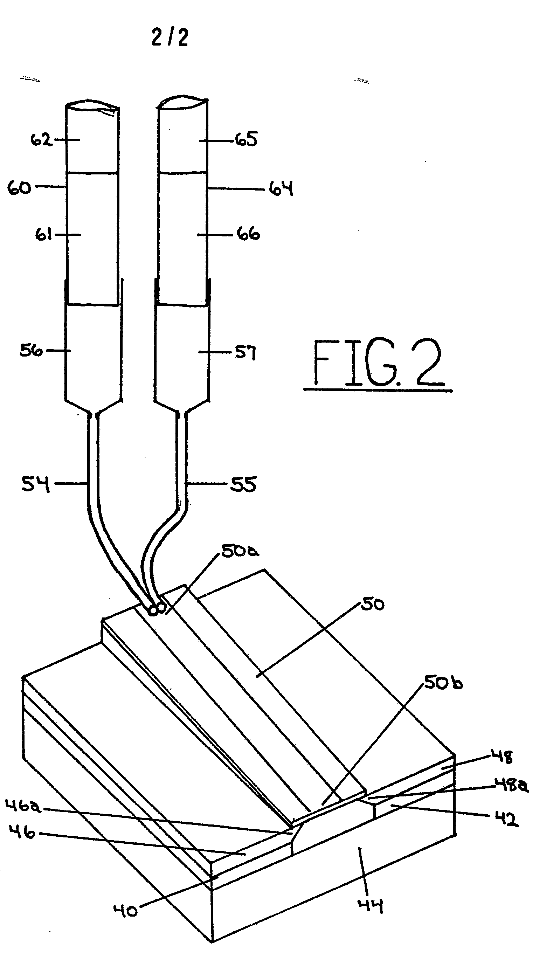

Another embodiment of an apparatus in accordance with this invention is depicted in Figure 2. The apparatus shown in Figure 2 utilizes a magnet pair and substrate similar to that shown and described in Figure 1. A pair of magnets, 40 and 42 are positioned on a magnetic return 44 and have pole pieces, 46 and 48, respectively, associated with them. The pole pieces have leading edges, 46a and 48a, respectively, which are separated by a narrow air gap so as to produce an intense field having a very high gradient in the air gap between them. This gradient · is in a plane transverse to the pole piece edges and has a downwardly directed component which magnetically collects particles onto a substrate 50 positioned above the magnets. The apparatus in Figure 2 is shown having dual reservoirs, 56 and 57, and feed tubes, 54 and 55, respectively, to provide separate reservoir and dispensing means for passing a particle-containing fluid across a substrate, and separate reservoir and dispensing means for passing a wash solvent across the substrate. A first reservoir 56 is shown having a tube 54 extending from the bottom of the reservoir 56 to the receiving end 50a of the substrate. Particle-containing fluid in reservoir 56 flows through tube 54 and onto the substrate 50 by positive displacement exerted from piston 60, which in turn is driven by a motor or pump, such as a positive displacement pump, not shown in the Figure.

The piston 60 may comprise a first section 61 extending into the reservoir and being detachable from a second section, permanently mounted piston 62. The detachable section of the piston 61 may be disposable, and so replacable for each particle-containing fluid sample to be run in the apparatus. This prevents contamination from one sample to the next. Likewise, the reservoir 56 may also be disposable along with tubing 54 to prevent sample-to-sample contamination. In one embodiment of this apparatus, the detachable portion of the piston 61 and the reservoir 56 comprise a disposable syringe that may be filled with particle-containing fluid, inserted into the apparatus, depleted of fluid by the action of permanently mounted piston section 62 and discarded after use.

Once piston 60 has extended fully downward into reservoir 56 and provided a positive displacement of the fluid in the reservoir into tube 54 and onto the substrate 50, a second piston 64, which may also have a permanent segment 65 and a detachable segment 66 is inserted into the second reservoir 57 having a wash solvent therein. The wash solvent is then positively displaced through tubing 55 and onto the substrate 50. The fluid is collected at the bottom of the substrate through a collection tube, not shown, which empties into a collection receptacle or collected directly into a collection receptacle, also not shown in the Figure.

By using this dual dispensation method, the operator may insert reservoirs 56 and 57, containing a particle-containing fluid and a wash solvent, respectively, and, having started the apparatus, the apparatus will continue its full operation without additional operator interaction or supervision.

From the foregoing it will be seen that a useful and reliable apparatus for measuring the relative concentration of particles of different sizes has been provided. This apparatus may be useful for either magnetically susceptible particles or for electrically polarizable particles, and is especially useful for measuring the relative concentrations of particles in a fluid a such as engine oil.

While the invention has been described with particular reference to the measurement of particles in engine oil, it will be understood that it is not so limited and that it may be used to monitor and measure the concentration rate of change of concentration, concentration ratios, etc.

of any magnetically susceptible or electrically polarizable particle in a fluid medium.

CLAIMS:
--------

1.      An apparatus for the separation of particles from
a fluid medium (22) in accordance with particle size and
for use with a magnet (10,12) providing an inhomogeneous
field, which apparatus comprises a substrate (20)
positioned in the magnetic field and supporting fluid
flow of the particle-containing fluid over a surface
thereof and receiving particles from the fluid thereon,
and tubing (24) for transporting said fluid to said
substrate, characterized by: a sealed reservoir (26)
disposed anterior to said tubing (24), which reservoir
contains a predetermined amount of said particle-
containing fluid, and means, optionally piston means,
for positively transfering said particle-containing fluid
into said tubing and thereonto said substrate.

2.      The apparatus in accordance with Claim 1 wherein
said piston (60) has a detachable segment (61) and a
permanently mounted segment (62).

3.      The apparatus in accordance with Claim 1 wherein
said reservoir (56) and said detachable piston segment
(61) comprise a syringe.

4.      The apparatus in accordance with Claim 1 wherein
said apparatus further comprises a second sealed reservoir
(57) disposed anterior to tubing (55), which reservoir
contains a predetermined amount of wash solvent and
second piston means (64) for positively transferring said
wash solvent into said tubing (55) and thereonto said
substrate (50).

5.     The apparatus in accordance with Claim 4 wherein said piston has a detachable segment (66) and a permanently mounted segment (65).

6.     The apparatus in accordance with Claim 5 wherein said second reservoir (57) and said detachable piston segment (66) comprise a syringe.

7.     The apparatus in accordance with Claim 4 wherein a positive-displacement pump automatically inserts a piston (60) into said reservoir (56) containing said particle-containing fluid and after which inserts a second piston (64) into said second reservoir (57) which contains said predetermined amount of wash solvent.

FIG.1

1/2

0196205

2/2

FIG.2